# EUROPEAN PATENT SPECIFICATION

(11) **EP 3 760 144 B1**
(45) Date of publication and mention of the grant of the patent: **01.10.2025**
(21) Application number: 20172110.7
(22) Date of filing: 29.04.2020
(51) Int. Cl.: A61B 17/34

(54) **TUBULAR ELEMENT FOR MEDICAL USE**
RÖHRENFÖRMIGES ELEMENT ZUR MEDIZINISCHEN VERWENDUNG
ÉLÉMENT TUBULAIRE POUR UTILISATION MÉDICALE

(30) Priority: 04.07.2019 IT 201900010875
(43) Date of publication of application: 06.01.2021
(73) Proprietor: Fiab S.P.A., 50039 Vicchio (IT)
(72) Inventor: VETTORI, Paolo, 50038 Scarperia e San Pietro (Firenze) (IT); CALABRO', Alberto, 50139 Firenze (IT); FASANO, Antonio, 50139 Firenze (IT)
(74) Representative: Milli, Simone

(56) References cited:
- EP-A1- 3 056 238
- WO-A1-2017/053288
- US-A- 5 752 939
- US-A1- 2008 108 974

## Description

This invention relates to the technical sector of devices for medical use.

In particular, the invention relates to a tubular element which can be used in surgical and out-patient operations which involve the insertion and use of probes, catheters or cannulas in a predetermined anatomical part of the body, for example blood vessels and heart cavities.

Generally speaking, the tubular elements designed to be inserted in a patient's body are delicate medical devices and designed for an equally delicate medical treatment and care purpose and therefore require particular attention in their design.

In particular, it is necessary for these elements to be made of biocompatible materials and with shapes and dimensions suitably designed to be used without risks for the patient.

It is also necessary for these devices to have structural features such as to allow the movement during use without creating risks for the health of the patient.

In this context, all the prior art solutions are subject to drawbacks and problems which sometimes render the above-mentioned tubular elements for medical use not very easy to use in certain specific situations or for particular technical reasons.

In particular, the prior art devices have ends configured to favour the insertion of the tubular element inside the human body.

For example, if one takes into consideration the specific case of percutaneous cannulas which are used for extracting components of medical devices implanted in heart chambers, the prior art devices have a pointed end which is able not only to facilitate the insertion, but also to promote the detachment of the components from the walls where they are implanted and in which they can have been at least partly incorporated following an adhesion/calcification process.

The tips of known type have a single operating angle which makes them suitably pointed to allow the separation of the components from the walls in question, but they are therefore generally disadvantageous since they are fragile and subject to the risk of damage due to the hardness of the structures on which they operate.

Examples of tubular catheters for surgical and out-patient operations to be inserted in blood vessels are described in patent documents EP 3056238 A1, US 2008/0108974 A1, WO 2017/053288 and US 5752939.

However, this catheter, although it has a marker which is able to prevent it from breaking, is not always detectable over its entire length since it depends on how it will be inserted inside the body of the patient.

In this context, the technical purpose which forms the basis of this invention is to provide a tubular element for medical use which overcomes at least some of the above-mentioned drawbacks of the prior art.

In particular, the aim of the invention is to provide a tubular element for medical use which is able to maintain a high degree of efficiency and ease of use, guaranteeing at the same time the integrity and solidity of the element, in particular during its movement inside the body of a patient.

The technical purpose indicated and the aims specified are substantially achieved by a tubular element for medial use comprising the technical features described in one or more of the appended claims.

The invention is defined in the appended claims and describes a tubular element for medical use which has a main body, a distal end and an intermediate portion.

The main body extends along a main axis of extension.

The distal end is configured for promoting the insertion of the subcutaneous element in a body, preferably inside a cardiovascular conduit, and has a front edge inclined relative to the main axis of extension to form a first operating angle.

The intermediate portion is configured for connecting the distal end to the main body and has an intermediate front edge inclined relative to the main axis of extension to form a second operating angle different from the first operating angle.

Advantageously, the tubular element described here is equipped with a tip which has along its axial extension at least two different operating angles; in this way, the distal end may have an angle such as to guarantee the stability, thus avoiding the risk of breakage or damage, whilst the intermediate portion, which connects the tip with the main body, maintains a different operating angle which facilitates the insertion inside the body of the patient. The at least double angle also provides a sort of "shoulder" which is particularly suitable for attaching to the above-mentioned calcified tissue concretions which immobilise the devices to be explanted.

The invention also relates to a percutaneous extrusion cannula configured for the complete or partial extraction of medical devices, both subcutaneous (for example of the neurological type) and from a cardiac chamber.

The cannula comprises a tubular element according to the invention.

The dependent claims relate to different embodiments of the invention.

Further features and advantages of this invention are more apparent in the detailed description below, with reference to a preferred, non-restricting, embodiment of a tubular element for medical use as illustrated in the accompanying drawings, in which:

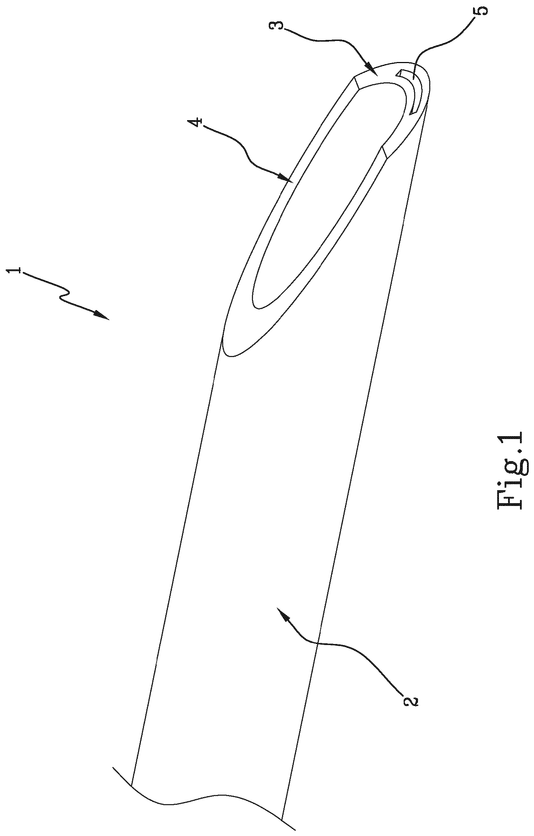

- Figure 1 is a perspective view of the end of the tubular element according to the invention designed to be inserted in the body of a patient;

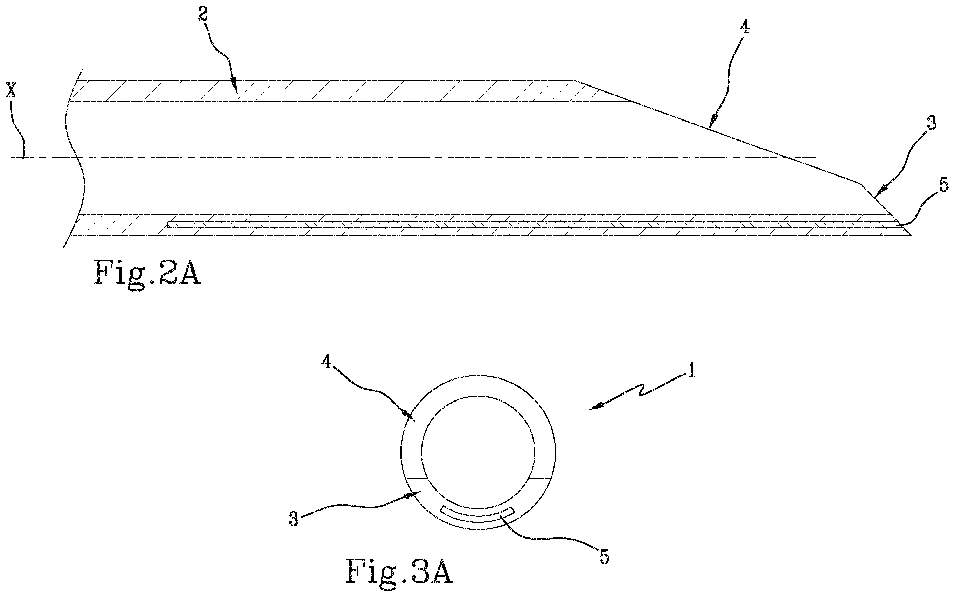

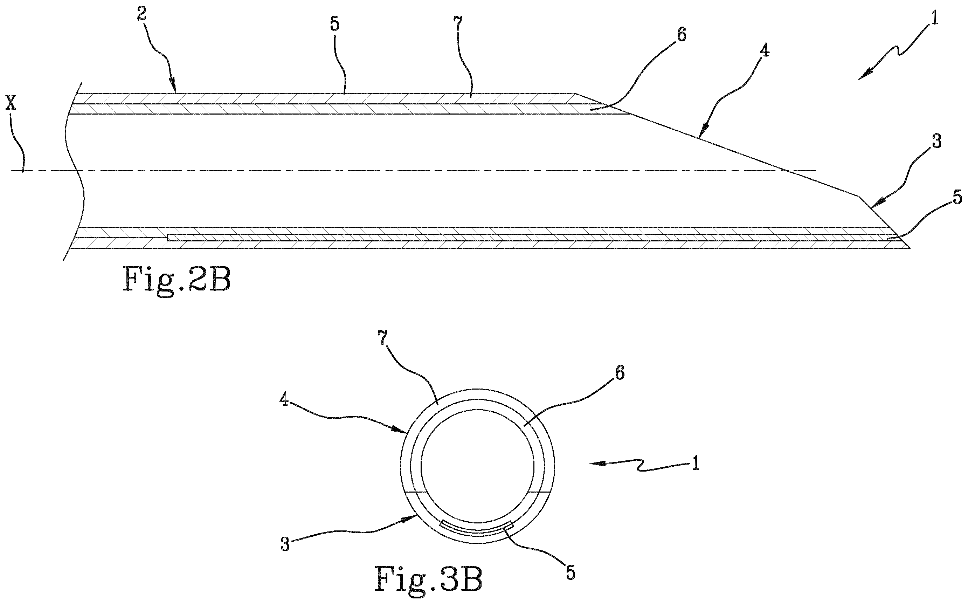

- Figures 2A-B are axial cross sections of the end of Figure 1, according to two possible embodiments;
- Figures 3A-B are radial cross sections of the tubular element in accordance with the different embodiments, respectively, of Figure 2A-B. In the accompanying drawings, the numeral 1 denotes in general a tubular element for medical use according to the invention, hereinafter referred to as element 1.

Hereinafter, by way of a non-limiting example, the term tubular element 1 for medical use will be used to refer, for example, to probes, catheters and cannulas which can be used during medical operations.

In particular, the element 1 has a main body 2, a distal end 3 and an intermediate portion 4.

The main body 2 has a main axis of extension "X" and defines a gripping portion, preferably at a proximal end of the element 1, by which a user can handle the element 1, in particular for inserting it into or extracting it from the body of a patient.

The distal end 3 is configured to promote the insertion and the movement of the subcutaneous element 1, preferably inside a cardiovascular conduit. For this reason, for the purpose of the invention, the distal and proximal terms are used with reference to a configuration of use of the element wherein the distal end 3 is the end designed to be inserted into the body of the patient, whilst the proximal end is the end designed to remain outside the body of the patient and preferably allow the retaining and the manipulating of the element 1 by the user.

More in detail, the distal end 3 has a front edge inclined relative to the main axis of extension "X", in such a way as to form a first operating angle α.

In other words, the operating angle defines the angular amplitude adopted by the element 1 at the distal end 3.

In particular, the first operating angle α is between 40° and 50°, preferably 45°.

Consequently, the distal end 3 has a pointed shape (when necessary suitably shaped by thermoforming), that is to say, it defines a tip with which it is possible to engage any concretions of any type which obstruct the extraction of the devices to be removed.

This function is particularly important if the element 1 is used to perform operations for extracting electro-catheters/catheters implanted in heart cavities.

In effect, in this particular context, the tip makes it possible to detach the sheath (constituting the outer covering of the electro-catheters themselves) and/or the metallic parts (electrodes) from the cardiovascular conduits in which they are inserted and in which they could have been positioned following processes of adherence and calcification, operating in fact like a chisel which separates the sheath from the walls, substantially cutting the concretions which have at least partly closed around the cavity. Preferably, the distal end 3 has an axial length of between 0.5 mm and 5 mm.

The intermediate portion 4 performs, on the other hand, a function of connecting between the distal end 3 and the main body 2.

Moreover, the intermediate portion 4 also has a front edge inclined relative to the main axis of extension "X", in such a way as to form a second operating angle β, which is different to the first operating angle α.

In particular, the second operating angle β is between 10° and 30°, preferably 20°.

Preferably, the intermediate portion 4 has an axial length of between 1.5 mm and 25 mm.

In other words, the part of the element 1 designed to promote the insertion into the body of a patient has two different operating angles α, β.

Advantageously, the invention achieves the preset aims overcoming the drawbacks of the prior art by providing the user with a tubular element for medical use wherein the synergic effect provided by the simultaneous presence of two different operating angles α, β makes it possible to better use the advantages provided by both without the relative drawbacks.

In particular, the first operating angle α, which is preferably greater in size, provides the distal end 3 with sufficient strength to allow the element 1 to operate correctly without being damaged, whilst the second operating angle β, which is preferably smaller in size, guarantees ease of insertion, allowing an optimum manoeuvrability as well as a greater effectiveness in the cutting of the concretions.

The element 1 also comprises a marker 5, that is to say, a substance different from the materials which make the rest of the element 1 and which can be detected by suitable detection systems.

In particular, the marker 5 may comprise at least one between: colouring agent, radio-opaque agent, opaque agent, magnetising agent, fluorescent agent, luminescent agent.

The marker 5 is applied at least at an angular portion of the side wall of the tubular element 1 which extends from the distal end 3 to at least a portion of the main body 2.

In other words, the marker 5 is applied in such a way as to extend at least partly along the main body 2 as well as on the part of the element 1 which defines the tip, that is, the distal end 3 and the intermediate portion 4. Preferably, the marker 5 is applied at an angular portion of the side wall of the element 1 which extends on the entire main body 2.

In other words, the entire element 1, therefore the entire main body 2, the distal end 3 and the intermediate portion 4, has the marker 5 applied along its entire length, in such a way as to make its entire profile detectable, irrespective of the length for which it will be inserted inside the body of the patient.

Preferably, the marker 5 is at least partly incorporated in the side wall, that is to say, the marker 5 is applied inside the side wall of the element 1.

According to a possible embodiment, not illustrated in the accompanying drawings, the element 1 comprises a first marker 5 applied at the distal end 3, a second marker 5 applied at the intermediate portion 4 and a third marker 5 applied at the main body 2.

These markers 5 may have different detecting characteristics, that is to say, they are susceptible to generate different measuring signals which allow them to be distinguished in a clear manner.

In this way it is possible to detect different specific signals for the various portions which make up the element 1, thus obtaining detailed information which makes it possible to define in a clear and immediate manner the actual position of the element 1 inside the body of the patient.

Advantageously, the presence of the marker applied not only on the tip of the element 1, but at least partly also on its main body 2 makes it possible to obtain during use an overall and immediate view of the actual position of the element 1 and of its arrangement inside the body of the patient.

The element 1 also comprises an inner tubular layer 6 and an outer tubular layer 7. The presence of several layers in other embodiments can be expected.

The inner tubular layer 6 defines an inner lateral surface of the element 1. The outer tubular layer 7 is, on the other hand, fitted around the inner tubular layer 6 and defines an outer side surface of the element 1.

In other words, the element 1 has a side wall made by radially superposing several tubular layers 6, 7.

According to a preferred embodiment, the inner tubular layer 6 and the outer tubular layer 7 are preferably made using different materials.

In particular, the materials which can be used can be biocompatible plastic materials suitably selected in particular according to their flexibility.

By way of a non-limiting example, the tubular layers 6, 7 may be made of Polycarbonate (PC), Polypropylene (PP), Polyethylene (PET), Polyacetal resin (POM), Polyamide (PA), Polyether ether ketone (PEEK), Polytetrafluoroethylene (PTFE).

It is therefore possible to make the element 1 using materials which have different elastic characteristics, flexibility/twisting in particular, in such a way as to modulate in a precise and accurate manner the mechanical properties in particular, as a function of the particular operating context in which the element 1 must be used.

Moreover, the inner tubular layer 6 may have a different thickness to that of the outer tubular layer 7.

In this way, the difference in thickness also allows the overall rigidity of the element 1 to be modulated.

In particular, the inner tubular layer 6 has a radial thickness of between 0.10 mm and 0.25 mm, whilst the outer tubular layer 7 has a radial thickness of between 0.10 mm and 0.25 mm.

It should be noted, therefore, that the element may have tubular layers 6, 7 made of different materials and/or with different radial thicknesses.

In particular, the inner tubular layer 6 and/or the outer tubular layer 7 have a radial thickness which may vary and be suitably modulated along the main axis of extension "X" of the tubular element 1.

In other words, it is possible that the element 1 has axial portions wherein the inner tubular layer 6 and the outer tubular layer 7 have radial thicknesses which vary, giving the element overall mechanical properties which vary as a function of the radial thicknesses.

Similarly, the material with which the inner tubular layer 2 and/or the outer tubular layer 3 are made may also be modified along at least some portions of the total length of the element 1.

The element 1 may also comprise at least one intermediate tubular layer, not illustrated in the accompanying drawings, interposed between the inner tubular layer 6 and the outer tubular layer 7.

In other words, it is possible to further improve the mechanical properties of the element 1 introducing further tubular layers, the radial thickness and material of which can be suitably selected so as to modify the overall rigidity of the element 1.

It is also possible to make different portions of the element 1 using different combinations of materials and/or thicknesses for the various tubular layers 6, 7 which make it up, in such a way as to locally modulate the rigidity and/or other mechanical properties of the element.

This aspect is particularly important with reference to the making of the distal end 3, since it is possible to further increase its resistance and its efficiency of use, suitably modulating the type of materials used for making it and their radial thickness.

In this context, the marker 5 is preferably interposed between the inner tubular layer 6 and the outer tubular layer 7.

Advantageously, the presence of several concentric tubular layers made of different materials and/or thicknesses allows the mechanical properties of the element to be modified in a precise and accurate manner, thus allowing elements 1 to be made specially designed to operate in specific situations.

For example, one of these concentric layers (typically the outermost one and/or the innermost one) may be identified in a suitable coating (hydrophilic, fluoropolymeric, etc.) with biomedical grade materials designed to minimise the possible friction which the element 1 may encounter inside the blood vessel, that is, of another similar cannula.

The invention also relates to a percutaneous extrusion cannula configured for the complete or partial extraction of medical devices, both subcutaneous (for example of the neurological type) and from a cardiac chamber.

The cannula comprises a tubular element as claimed.

## Claims

1. A tubular element (1) for medical use having:
- a main body (2) extending along a main axis of extension (X);
- a distal end (3) having a front edge inclined relative to the main axis of extension (X) in such a way as to form a first operating angle (α);
- an intermediate portion (4) configured to join the distal end (3) to the main body (2) and having a front edge inclined with respect to the main axis of extension (X) in such a way as to form a second operating angle (β) which is different from said first operating angle (α);
**characterised in that** it comprises
a marker (5) applied at least at an angular portion of the side wall of the entire main body (2) of the tubular element (1) which extends from the distal end (3) to at least a portion of the main body (2);
said operating angle (α) being the angular amplitude adopted by the tubular element at said distal end (3);said marker (5) being applied from the tip of the side wall of the tubular element over its entire length entirely in said tubular element (1), therefore the entire said main body (2), said distal end (3) and said intermediate portion (4) have the marker (5) applied along their entire respective length, in such a way as to make it possible to detect the entire profile of said marker (5), irrespective of the length for which it will be inserted inside the body of the patient.

2. The element (1) according to claim 1, wherein the first operating angle (α) is between 40° and 50°, preferably said first operating angle (α) being equal to 45°, and the second operating angle (β) is between 10° and 30°, preferably said second angle (β) being equal to 20°.

3. The element (1) according to claim 1 or 2, wherein the distal end (3) has an axial length of between 0.5 mm and 5 mm and wherein the intermediate portion (4) has an axial length of between 2.5 mm and 25 mm.

4. The element (1) according to any one of the preceding claims, wherein the marker (5) is interposed between an inner tubular layer (6) and an outer tubular layer (7) of said tubular element (1), said inner tubular layer (6) being designed to define an inner side surface of said tubular element (1) and said outer tubular layer (7), fitted around said inner tubular layer (6), being designed to define an outer side surface of said tubular element (1).

5. An element (1) according to claim 4, wherein the marker (5) is at least partly incorporated in the side wall.

6. The element (1) according to any one of claims 4 or 5, wherein the marker (5) comprises at least one between: colouring agent, radio-opaque agent, opaque agent, magnetising agent, fluorescent agent, luminescent agent.

7. The element (1) according to any one of claims 1-6, comprising:
- a first marker (5) applied at the distal end (3);
- a second marker (5) applied at the intermediate portion (4);
- a third marker (5) applied at the main body (2).

8. The element (1) according to any one of claims 4-7, wherein the inner tubular layer (6) and the outer tubular layer (7) are made of different materials.

9. The element (1) according to any one of claims 4 to 8, wherein a radial thickness of the inner tubular layer (6) is different from a radial thickness of the outer tubular layer (7), preferably said inner tubular layer (6) having a radial thickness of between 0.10 mm and 0.25 mm and said outer tubular layer (7) having a radial thickness of between 0.10 mm and 0.25 mm.

10. The element (1) according to any one of claims 4 to 9, comprising at least an intermediate tubular layer interposed between the inner tubular layer (6) and the outer tubular layer (7).

11. The element (1) according to any one of claims 4 to 10, wherein the marker (5) is interposed between the inner tubular layer (6) and the outer tubular layer (7).

12. The element (1) according to any one of claims 4 to 11, wherein the inner tubular layer (6) and/or the outer tubular layer (7) have a radial thickness varying along a main axis of extension (X) of the tubular element.

13. A percutaneous extraction cannula configured for the entire or partial extraction of medical devices implanted below the skin or in organic cavities, for example in heart cavities, comprising a tubular element (1) according to any one of the claims 1-12.

## Patentansprüche

1. Röhrenförmiges Element (1) zur medizinischen Verwendung, aufweisend:
- ein Hauptkörper (2), der sich entlang einer Haupterstreckungsachse (X) erstreckt;
- ein distales Ende (3) mit einer zur Haupterstreckungsachse (X) geneigter Vorderkante, um einen ersten Arbeitswinkel (α) zu formen;
- ein Zwischenabschnitt (4), der ausgelegt ist, um das distale Ende (3) und den Hauptkörper (2) zusammenzufügen und mit einer in Bezug auf die Haupterstreckungsachse (X) geneigter Vorderkante, um einen zweiten Arbeitswinkel (β), der sich vom ersten Arbeitswinkel (α) unterscheidet, zu formen;
**dadurch gekennzeichnet, dass** es Folgendes umfasst
einen Marker (5), der zumindest an einem Winkelabschnitt der Seitenwand des gesamten Hauptkörpers (2) des röhrenförmigen Elements (1), das sich vom distalen Ende (3) bis mindestens zu einem Abschnitt des Hauptkörpers (2) erstreckt, angebracht ist;
wobei der Arbeitswinkel (α) die Winkelamplitude ist, die das röhrenförmige Element an seinem distalen Ende (3) einnimmt; wobei der Marker (5) von der Oberkante der Seitenwand des röhrenförmigen Elements über dessen gesamte Länge vollständig in dem röhrenförmigen Element (1) angebracht ist, daher ist am gesamten Hauptkörper (2), am distalen Ende (3) und am Zwischenabschnitt (4) über ihre gesamte Länge der Marker (5) angebracht, um das gesamte Profil des Markers (5), ungeachtet der Länge, für die er in den Körper des Patienten eingeführt wird, zu erkennen.

2. Element (1) nach Anspruch 1, wobei der erste Arbeitswinkel (α) zwischen 40° und 50° liegt, vorzugsweise ist der erste Arbeitswinkel (α) gleich 45°, und der zweite Arbeitswinkel (β) liegt zwischen 10° und 30°, vorzugsweise ist der zweite Arbeitswinkel (β) gleich 20°.

3. Element (1) nach Anspruch 1 oder 2, wobei das distale Ende (3) eine axiale Länge zwischen 0,5 mm und 5 mm aufweist und wobei der Zwischenabschnitt (4) eine axiale Länge zwischen 2,5 mm und 25 mm aufweist.

4. Element (1) nach einem der vorhergehenden Ansprüche, wobei der Marker (5) zwischen der inneren röhrenförmigen Schicht (6) und der äußeren röhrenförmigen Schicht (7) des röhrenförmigen Elements (1) eingefügt ist, wobei die innere röhrenförmige Schicht (6) dazu ausgestaltet ist, die innere Seitenfläche des röhrenförmigen Elements (1) und die äußere röhrenförmige Schicht (7) zu definieren, und rund um die innere röhrenförmige Schicht (6), dazu ausgestaltet, eine äußere Seitenfläche des röhrenförmigen Elements (1) zu definieren, angebracht.

5. Element (1) nach Anspruch 4, wobei der Marker (5) zumindest teilweise in die Seitenwand integriert ist.

6. Element (1) nach einem der Ansprüche 4 oder 5, wobei der Marker (5) mindestens eines der folgenden Elemente umfasst: Farbstoff, strahlenundurchlässiges Mittel, undurchsichtiges Mittel, magnetisierendes Mittel, fluoreszierendes Mittel, lumineszierendes Mittel.

7. Element (1) nach einem der Ansprüche 1-6, umfassend:
- ein am distalen Ende (3) angebrachter erster Marker (5);
- ein am Zwischenabschnitt (4) angebrachter zweiter Marker (5);
- ein am Hauptkörper (2) angebrachter dritter Marker (5) .

8. Element (1) nach einem der Ansprüche 4-7, wobei die innere röhrenförmige Schicht (6) und die äußere röhrenförmige Schicht (7) aus unterschiedlichen Materialien sind.

9. Element (1) nach einem der Ansprüche 4 bis 8, wobei die radiale Dicke der inneren röhrenförmigen Schicht (6) sich von der radiale Dicke der äußeren röhrenförmige Schicht (7) unterscheidet, wobei vorzugsweise die innere röhrenförmige Schicht (6) eine radiale Dicke zwischen 0,10 mm und 0,25 mm und die äußere röhrenförmige Schicht (7) eine radiale Dicke zwischen 0,10 mm und 0,25 mm aufweist.

10. Element (1) nach einem der Ansprüche 4 bis 9, umfassend mindestens eine röhrenförmige Zwischenschicht, die zwischen der inneren röhrenförmigen Schicht (6) und der äußeren röhrenförmigen Schicht (7) eingefügt ist.

11. Element (1) nach einem der Ansprüche 4 bis 10, wobei der Marker (5) zwischen der inneren röhrenförmigen Schicht (6) und der äußeren röhrenförmigen Schicht (7) eingefügt ist.

12. Element (1) nach einem der Ansprüche 4 bis 11, wobei die innere röhrenförmige Schicht (6) und/oder die äußere röhrenförmige Schicht (7) eine radiale Dicke aufweisen, die entlang einer Haupterstreckungsachse (X) des röhrenförmigen Elements variiert.

13. Perkutane Extraktionskanüle, ausgelegt zur vollständigen oder teilweisen Extraktion von unter der Haut oder in organischen Hohlräumen implantierten medizinischen Vorrichtungen, beispielsweise in Herzkammern, umfassend ein röhrenförmiges Element (1) nach einem der Ansprüche 1-12.

## Revendications

1. Élément tubulaire (1) pour utilisation médicale, comportant :
- un corps principal (2) s'étendant le long d'un axe principal d'extension (X) ;
- une extrémité distale (3) comportant un bord avant incliné par rapport à l'axe principal d'extension (X) de manière à former un premier angle de fonctionnement (α);
- une partie intermédiaire (4) configurée pour joindre l'extrémité distale (3) au corps principal (2) et comportant un bord avant incliné par rapport à l'axe principal d'extension (X) de manière à former un second angle de fonctionnement (β) différent dudit premier angle de fonctionnement (α) ;
**caractérisé en ce qu'**il comprend
un marqueur (5) appliqué au moins à une partie angulaire de la paroi latérale de l'ensemble du corps principal (2) de l'élément tubulaire (1) qui s'étend de l'extrémité distale (3) à au moins une partie du corps principal (2) ;
ledit angle de fonctionnement (α) étant l'amplitude angulaire adoptée par l'élément tubulaire à ladite extrémité distale (3) ; ledit marqueur (5) étant appliqué à partir de la pointe de la paroi latérale de l'élément tubulaire sur toute sa longueur entièrement dans ledit élément tubulaire (1), par conséquent, la totalité dudit corps principal (2), ladite extrémité distale (3) et ladite partie intermédiaire (4) comportent le marqueur (5) appliqué sur toute leur longueur respective, de manière à permettre de détecter l'ensemble du profil dudit marqueur (5), quelle que soit la longueur pour laquelle il sera inséré à l'intérieur du corps du patient.

2. Élément (1) selon la revendication 1, dans lequel le premier angle de fonctionnement (α) est compris entre 40 et 50°, de préférence ledit premier angle de fonctionnement (α) étant égal à 45°, et le second angle de fonctionnement (β) est compris entre 10 et 30°, de préférence ledit second angle (β) étant égal à 20°.

3. Élément (1) selon la revendication 1 ou 2, dans lequel l'extrémité distale (3) a une longueur axiale comprise entre 0,5 et 5 mm et dans lequel la partie intermédiaire (4) a une longueur axiale comprise entre 2,5 et 25 mm.

4. Élément (1) selon l'une quelconque des revendications précédentes, dans lequel le marqueur (5) est interposé entre une couche tubulaire interne (6) et une couche tubulaire externe (7) dudit élément tubulaire (1), ladite couche tubulaire interne (6) étant conçue pour définir une surface latérale interne dudit élément tubulaire (1) et ladite couche tubulaire externe (7), ajustée autour de ladite couche tubulaire interne (6), étant conçue pour définir une surface latérale externe dudit élément tubulaire (1).

5. Élément (1) selon la revendication 4, dans lequel le marqueur (5) est au moins partiellement incorporé dans la paroi latérale.

6. Élément (1) selon l'une quelconque des revendications 4 ou 5, dans lequel le marqueur (5) comprend au moins un agent parmi : un agent colorant, un agent radio-opaque, un agent opaque, un agent magnétisant, un agent fluorescent, un agent luminescent.

7. Élément (1) selon l'une quelconque des revendications 1-6, comprenant :
- un premier marqueur (5) appliqué à l'extrémité distale (3) ;
- un deuxième marqueur (5) appliqué à la partie intermédiaire (4) ;
- un troisième marqueur (5) appliqué au corps principal (2).

8. Élément (1) selon l'une quelconque des revendications 4-7, dans lequel la couche tubulaire interne (6) et la couche tubulaire externe (7) sont constituées de matériaux différents.

9. Élément (1) selon l'une quelconque des revendications 4 à 8, dans lequel une épaisseur radiale de la couche tubulaire interne (6) est différente d'une épaisseur radiale de la couche tubulaire externe (7), de préférence ladite couche tubulaire interne (6) ayant une épaisseur radiale comprise entre 0,10 et 0,25 mm et ladite couche tubulaire externe (7) ayant une épaisseur radiale comprise entre 0,10 et 0,25 mm.

10. Élément (1) selon l'une quelconque des revendications 4 à 9, comprenant au moins une couche tubulaire intermédiaire interposée entre la couche tubulaire interne (6) et la couche tubulaire externe (7) .

11. Élément (1) selon l'une quelconque des revendications 4 à 10, dans lequel le marqueur (5) est interposé entre la couche tubulaire interne (6) et la couche tubulaire externe (7).

12. Élément (1) selon l'une quelconque des revendications 4 à 11, dans lequel la couche tubulaire interne (6) et/ou la couche tubulaire externe (7) ont une épaisseur radiale variant le long d'un axe principal d'extension (X) de l'élément tubulaire.

13. Canule d'extraction percutanée, configurée pour l'extraction totale ou partielle de dispositifs médicaux implantés sous la peau ou dans des cavités organiques, par exemple dans des cavités cardiaques, comprenant un élément tubulaire (1) selon l'une quelconque des revendications 1-12.
